# EUROPEAN PATENT APPLICATION

(11) **EP 3 650 218 A2**
(43) Date of publication of application: **13.05.2020**
(21) Application number: 19207344.3
(22) Date of filing: 06.11.2019
(51) Int. Cl.: B32B 27/20, B32B 27/08, A61F 13/15, B65D 85/00

(54) **MULTILAYER SHEET WITH DIFFERENTIAL STRUCTURE**

(30) Priority: 07.11.2018 IT 201800010093
(71) Applicant: Plastik Textile S.p.a., 24061 Albano S. Alessandro (Bergamo) (IT)
(72) Inventor: CATTANEO, Gianangelo, I-24061 Albano Sant'Alessandro, BERGAMO (IT); MARINUCCI, Luca, I-24061 Albano Sant'Alessandro, BERGAMO (IT); CASTELLI, Simone, I-24061 Albano Sant'Alessandro, BERGAMO (IT)
(74) Representative: Croce, Valeria

(57) **Abstract**

The present patent application concerns a multilayer sheet, which is used in the packaging sector, as well as a process for the preparation thereof.

## Description

### State of the art

Every year about 270 million tons of crude oil, or 4% of the world production, is transformed into plastic and the resulting environmental impact, in view of the global growth in consumption, is not sustainable. By way of example, the massive use of polymer films for the packaging of consumer products, including single use packaging, is significant. Among these uses, one may point out the pouches for the packaging of sanitary napkins for women.

There is also a need to limit the use of cellulose for paper production, so as to limit the felling of trees for this purpose.

The object of the present invention is to provide a multilayer sheet that finds advantageous application as a substitute for the films currently in use for the packaging of products, as well as a valid alternative to paper.

Advantageously, the solution proposed here has a considerably reduced environmental impact with respect to the solutions available today.

A further requirement satisfied by the present solution is to have a siliconized film, for use, for example, in the packaging of sanitary napkins for women, which overcomes the drawbacks of those available today.

Women's sanitary napkins have a suitable shape to adhere to the inside of panties, where the surface of the napkin that is to adhere is provided with an adhesive to anchor itself to the fabric of the inside of the panties. Said anchoring of the structure of the napkin inside the panties is typically achieved by means of a layer of adhesive or tacky material: by way of example of the type D3347 of H.B. FULLER - SICAM. This tacky material must be kept covered, both to prevent it from adhering to foreign bodies, to reduce the evaporation of any solvents, and to avoid other chemical-physical phenomena that would otherwise affect the adhesive properties at the time of use. Said covering of the layer of tacky material must obviously be made with elements suitable to adhere thereto with minimum force, which makes it easy to remove said covering. However, this minimum adhesion force must be sufficient to ensure the stable positioning of this covering in order to enable it to fulfill its intended function.

The material pre-eminently used in this role of removable covering is silicone, generally applied on a strip of paper (siliconized paper) as a rigid support. With this solution, the structure of the napkin is freely housed within a containment pouch. Said pouch is intended as a packaging element for individual structures of disposable napkins. After being emptied of its contents, said pouch may be kept for placing therein the used napkin until it may be thrown away in a suitable place.

An evolution of the strip of siliconized paper to be removed before use is expressed by a version wherein said strip is anchored on one side, inside the pouch, by a second adhesive having a "tack" greater than the one existing on the other side, arranged to protect the adhesive material provided for anchoring inside the panties. This solution has the advantage of automatically removing the siliconized paper with the same maneuver of extracting the napkin from its pouch; however, it has the disadvantage of having a pouch made "bulky" by the presence of the siliconized paper glued thereto.

US patents US 5,181,610 and US 5,474,818 describe polyethylene pouches having on the interior thereof a surface treated with a silicone "coating" process.

US Patent 6,261,674 describes a two-layer polymer film, wherein the polymer material that makes up the first layer is different from the polymer material that makes up the second layer, so that the two layers have a different permeability to water.

Also known are the production techniques of polyethylene films, for the construction of the aforementioned containment pouches for the usual hygienic napkins, made by coextrusion of silicone film.

The production process of said polyethylene films for pouches used for the individual packaging of hygienic devices (siliconized pouch film) provides for the application, by means of flexographic printing, of a silicone monomer on one of the faces of the film.

This monomer is in solution with a catalyst susceptible to ultraviolet radiation (photoinitiator). Immediately after application on the film, this solution is then subjected to UV radiation to trigger the polymerization process.

Generally, at the end of production, the non-siliconized side shows a drastic decrease in the friction coefficient and ultimately also a low tendency to letting adhesive tapes adhere. The most reliable hypothesis after years of testing is that the polymerization is not complete at the end of printing and a portion of the monomer is transferred accidentally onto the opposite face while winding in reel (SETOFF phenomenon).
It is therefore strongly felt the need to provide economical and functional pouches, made of extremely flexible and thin, waterproof, opaque material, which overcome the drawbacks of pouches of the state of the art.

### Object of the invention

In a first object, the present invention describes a multilayer sheet comprising at least one microporous layer and at least one monolithic layer.

In a second object, a process for preparing the multilayer sheet of the invention is described.

In a third object, a film is described comprising the multilayer sheet of the invention on which a layer of nonstick material is applied.

The process for preparing the film represents a fourth object of the invention.

A packaging made from the multilayer sheet or the film described above represents further objects of the present invention.

A packaging represented by an envelope or a sachet or a pouch made with the multilayer sheet or the described film represent particular objects of the present invention.

A pouch made with the multilayer sheet or the film described represents a further particular objects of the present invention.

The use of the multilayer sheet or film for the packaging of food for writing and/or printing is a further object of the present invention.

### Brief description of the figures

Figure 1 represents a section of a sample multilayer sheet according to the present invention.
Figure 2 represents an example of a pouch for the containment of a sanitary napkin according to an object of the present invention.
Figure 3 represents a section of an exemplifying siliconized sheet according to the present invention.

### Detailed description of the invention

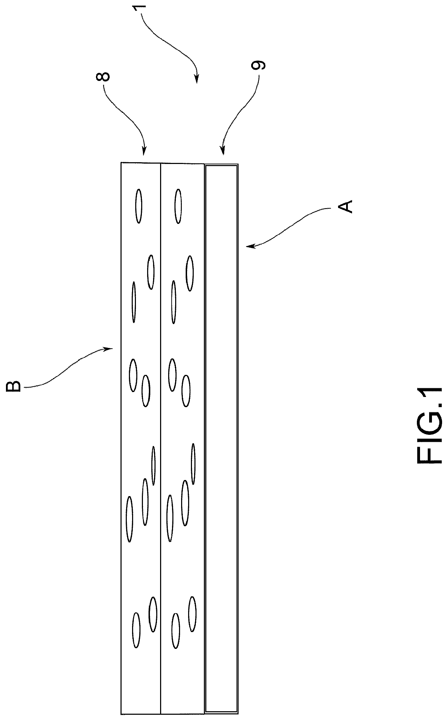

In accordance with a first object of the invention, a multilayer sheet 1 is described that comprises at least one microporous layer 8 and at least one monolithic layer 9, as for example shown in Figure 1.

For the purposes of the present invention, said microporous layer 8, thus defined in that it is characterized by a plurality of microcavities distributed in said matrix, comprises a polymer matrix in which are dispersed mineral fillers in a quantity of about 20-80% (w/w).

In particular, the polymer matrix of the microporous layer 8 is represented by a polymer chosen from the group which comprises polyolefins, selected for example from: linear low-density polyethylene (LLDPE), low-density polyethylene (LDPE), high-density polyethylene (HDPE), polypropylene (PP), or mixtures thereof, for example polyethylene, polypropylene, ethylene-propylene copolymers.

In a preferred aspect, the polymer matrix of the microporous layer 8 is represented by a polymer selected in the group comprising: linear low-density polyethylene (LLDPE), low-density polyethylene (LDPE), high-density polyethylene (HDPE).

In one embodiment, said fillers are preferably dispersed in a quantity of about 40-80% (w/w), preferably about 50-75% (w/w) and even more preferably of about 65-75% (w/w).

In particular, the mineral fillers are selected from the group comprising: calcium carbonate, talc, zeolites, kaolin, silica, magnesium carbonate, barium carbonate, magnesium sulphate, barium sulphate, calcium sulphate, aluminum hydroxide, magnesium hydroxide, zinc oxide, alumina, mica; or mixtures thereof.

In a preferred embodiment, said mineral fillers are represented by calcium carbonate.

For the purposes of the present invention, the mineral fillers of the microporous layer 8 have preferably an average particle size of about 1-5 µm, preferably about 1.5-2.5 µm and even more preferably of about 1.7 µm.

Smaller particle sizes do not allow an effective formation of micro-fractures, while with higher particle sizes said micro-fractures become excessive leading to breakage of the polymer matrix.

As for the monolithic layer 9, it comprises a polymer matrix.

In particular, the polymer matrix of the monolithic layer 9 is represented by a polymer selected from the group which comprises polyolefins, selected for example from the group that comprises: linear low-density polyethylene (LLDPE), low-density polyethylene (LDPE), high-density polyethylene (HDPE), polypropylene (PP), or mixtures thereof, for example polyethylene, polypropylene, ethylene-propylene copolymers.

In a preferred aspect, this polymer matrix of the monolithic layer 9 comprises: linear low-density polyethylene (LLDPE), possibly low-density polyethylene (LDPE), possibly polypropylene (PP) .

In an even more preferred aspect, this polymer matrix of the monolithic layer 9 is represented by: 0-20% low-density polyethylene (LDPE), 40-90% linear low-density polyethylene (LLDPE) and 0-30% polypropylene (PP).

According to an embodiment of the invention, the monolithic layer 9 may comprise mineral fillers.

In a preferred aspect of the invention, these mineral fillers are present in a quantity of about 0-15% (w/w), preferably about 8-15% and more preferably about 8-10% (w/w).

In particular, said mineral fillers are chosen from the group which comprises: calcium carbonate, talc, zeolites, kaolin, silica, magnesium carbonate, barium carbonate, magnesium sulfate, barium sulfate, calcium sulfate, aluminum hydroxide, magnesium hydroxide, zinc oxide, alumina, mica; or mixtures thereof.

For the purposes of the present invention, the mineral fillers of the monolithic layer 9 have preferably an average particle size of about 1-5 µm, preferably of about 1.5-2.5 µm and even more preferably of about 1.7 µm.

Smaller particle sizes do not allow an effective formation of micro-fractures, while with higher particle sizes said micro-fractures become excessive leading to breakage of the polymer matrix.

In a preferred aspect of the invention, the mineral fillers of the monolithic layer 9 are represented by calcium carbonate.

For the purposes of the present invention, the multilayer sheet 1 described may have a thickness of about 10-100 pm; in particular, according to the thickness, one or the other application will be preferable, as it will be detailed below.

According to the alternative embodiments of the invention, the multilayer sheet 1 may consist of six layers, or five, or three layers, wherein the monolithic and microporous layers alternate.

In a preferred embodiment, the multilayer sheet 1 of the invention comprises two layers, one of which is microporous 8 and one monolithic 9.

According to the embodiment of the multilayer sheet 1 of the invention comprising more than one microporous layer (8) and/or more than one monolithic layer 9, the only or one of said monolithic layers 9 is on the outside of said multilayer sheet (1) .

According to an embodiment of the invention, for example represented in Figure 1, the multilayer sheet 1 has an inner face A, which is represented by a monolithic layer, and an outer face B, which is represented by a microporous layer.

For the purposes of the present invention, the multilayer sheet 1 is a hybrid of the "breathable film" (the microporous layer) and a standard polyethylene film (monolithic layer).

For the purposes of the present invention, said microporous 8 and monolithic 9 layers of the multilayer sheet are preferably in a thickness ratio of about 3/4 and 1/4 or about 2/3 and 1/3, respectively.

Advantageously, said ratios increase the absorbency of the multilayer sheet, where a greater thickness of the or at least one microporous layer corresponds to a greater absorbency, while the presence of the monolithic layer, having a thickness of at least 1 µm, ensures the non-permeability of said multilayer sheet.

In accordance with a second object, a process for preparing the multilayer sheet 1 of the present invention is described.

In particular, this process comprises the steps of:
a)providing at least one first polymer matrix and at least one second polymer matrix;
b)coextruding said first and said second polymer matrix in the form of monolayers thus forming a multilayer sheet 1, where said multilayer sheet 1 is obtained by combining different hot layers inside the extruder.

For the purposes of the present invention, in step a) above, said at least first polymer matrix is intended for the preparation of a microporous layer.

According to a preferred aspect of the present invention, said microporous layer is the microporous layer 8 described above.

For the purposes of the present invention, in step a) above, said at least one second polymer matrix is intended for the preparation of a monolithic layer.

According to a preferred aspect of the present invention, said monolithic layer is the monolithic layer 9 described above.

The process of the present invention further comprises a stretching step of said first polymer matrix which, in a preferred aspect, is conducted longitudinally.

In particular, through the stretching step, an elongation of the polymer matrix is caused, while the mineral fillers contained therein, being substantially inert and rigid, do not undergo any elongation; this different behavior determines the formation of micro fractures in the polymer matrix right next to the filler particles, determining the formation of the microporous polymer matrix.

In a particular aspect of the process of the invention, said elongation is preferably achieved by passing through a stretching machine composed of a series of cylinders that rotate at progressively increasing tangential speeds.

More specifically, said cylinders are controlled thermally, so as to allow the polyolefins that make up said polymer matrix to reach a softening temperature that, for the polyethylenes, is between 50°C and 80°C; in this way, they may be easily stretched without excessive plasticity.

In one aspect of the present invention, the typical stretching ratio applied, where the stretching ratio is the ratio between the final speed of the cylinders and the initial speed before the stretching machine, is about 1.5 to 6.

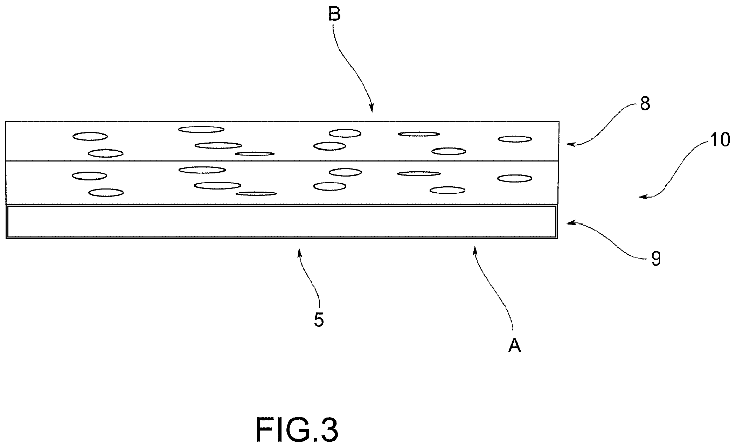

According to a third object of the invention, for example represented in Figure 3, the multilayer sheet 1 described above may further comprise a layer of nonstick material 5, thus obtaining a product referred to as film 10.

In a preferred aspect, said nonstick material is represented by silicone and said layer of nonstick material 5 is therefore a layer of silicone.

The process for preparing said film 10 is a fourth object of the present invention.

In particular, this process comprises the preparation of the multilayer sheet 1 as described above and comprises a further step of applying a layer of a nonstick material 5.

According to a preferred aspect of the invention, said nonstick layer 5 is applied on the monolithic layer 9, however, on the outer monolithic layer.

According to an even more preferred aspect of the invention, said nonstick layer 5 is represented by a layer of silicone material.

The application of said nonstick material 5 to said multilayer sheet 1 is obtained with methods known to the person skilled in the art, for example by flexographic techniques, or rotogravure.

Preferably, said nonstick material 5 is a silicone monomer applied in solution with a photoinitiator; in this way, after the application, said solution is subjected to UV radiation that triggers the polymerization process.

This allows that, even after winding in reels, the film 10 maintains its characteristics unaltered; in effect, said micro-fractures present in the microporous layer, by capillarity and at atmospheric pressure, allow the physical adsorption of the non-crosslinked monomer, avoiding its unwanted scattering on the opposite side of the same film 10.

In accordance with another object, the invention concerns a packaging made with the multilayer sheet 1 or with the film described above.

In a preferred aspect of the present invention, the multilayer sheet 1 of the present invention has a thickness of about 50-100 µm.

According to an embodiment of the invention, the multilayer sheet 1 finds a particular application in replacing sheets of paper in their traditional uses, such as, for example, sheets for writing in general, package wrapping paper, gift wrapping paper.

For this application, the multilayer sheet 1 of the invention has preferably a thickness of about 20-100 µm.

For these purposes, the monolithic layer 8 may have a thickness of 1 µm or more.

According to other embodiments of the invention, the multilayer sheet 1 is used for the production of food packaging, for example in the packaging of snacks, butchery products, cold cuts, dairy products, take-away food.

In accordance with a further particular object of the present invention, the film 10 described is used for making packaging for hygienic articles.

For example, such hygienic articles may be represented by sanitary napkins, for example for women.

For this purpose, the film 10 of the invention is preferably between 10 and 30 µm thick.

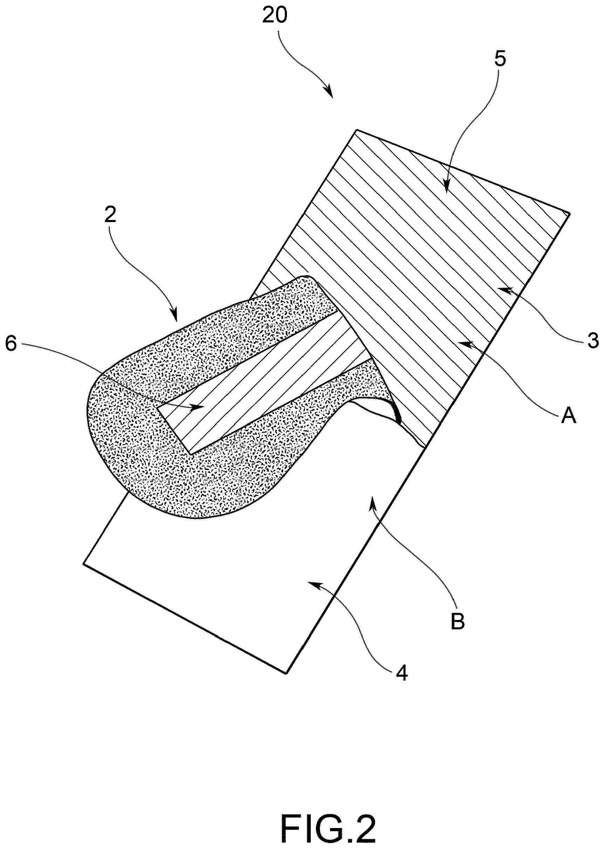

According to an embodiment of the invention, for example represented in Figure 2, the film 10 described is folded on itself, so as to form a packaging in the form of a pouch 20.

In particular, this pouch 20 comprises an outer side B, represented by the microporous layer 8 or by at least one of the microporous layers of the multilayer sheet 1, and an inner side A, represented by the monolithic layer 9 or at least one of the monolithic layers of the multilayer sheet.

As described above, the nonstick layer 5, preferably silicone, is applied to the monolithic layer 9.

For the purposes of the present invention, this nonstick silicone layer 5 has a preferred thickness of about 0.3-0.6 µm.

Said microporous layer 8 ensures that the residual nonstick material 5, after deposit on said face A, penetrates said layer, thus obviating the problem of films in use in the state of the art related to an unwanted redistribution of said nonstick material from face A to face B.

As shown in Figure 2, therefore, on said inner side A the sanitary napkin 2 is anchored by means of a layer of glue 6 applied to the sanitary napkin 2.

Advantageously, said nonstick layer 5 allows said sanitary napkin 2 to easily detach from said pouch 20, leaving on said sanitary napkin 2 a quantity of glue useful for an effective and functional application inside the panties and for the complete removal thereof.

As mentioned above, more generally, a package made with the multilayer sheet 1 or the film 10 described above represent further subject-matters of the present invention.

In particular, such packaging may consist of an envelope or a sachet or pouch; for example, such packaging may be used for the packaging of food.

More specifically, when used to make an envelope, the multilayer sheet 1 of the invention is preferably 20-70 µm thick.

According to a further subject-matter of the invention, the multilayer sheet 1 or the film 10 may be used for writing and/or printing, in place of paper.

### EXAMPLE 1

### Dart Impact Strength

A multilayer sheet according to the present invention and comprising a monolithic layer comprising a quantity of mineral fillers represented by calcium carbonate of about 10-15% (weight/weight) is subjected to impact resistance tests according to the ASTM D-1709 methodology (methodology A).
This standardized method allows the energy required to generate a film breakage under certain impact conditions of a dart (metal object with standardized geometry) in free fall to be generated. The energy is expressed as the weight of the dart falling from a specified height and resulting in the failure of 50% of the tested samples.
Method A uses a dart with a hemispherical head with a diameter of 38.10 ± 0.13 mm dropped from a height of 0.66 ± 0.01 m.

| | US 2017/129228 **(riv 18)** | **invention** |
|---|---|---|
| Resistance (12-10 g/sqm) | >50/70 g | 80-100 g |

From the aforesaid description, the advantages stated above offered by the present invention will be immediately evident to the person skilled in the art.

First of all, the multilayer sheet described comprises, with the same surface area, a drastically lower content of polymer matrix compared to a traditional polymer sheet, due to the volumes of mineral filler dispersed in the same.

This allows a reduced environmental impact.

Surprisingly, this advantage in no way detracts from the technical properties of the material's strength.

Moreover, the presence of a plurality of micro-cavities distributed in said polymer matrix makes the sheet, according to the present invention, particularly absorbent and as such suitable for printing and writing processes.

## Claims

1. A multilayer film (1) comprising at least two co-extruded layers: at least one microporous layer (8) and at least one monolithic layer (9) **characterized in that** said at least one microporous layer (8) comprises a polymer matrix in which mineral fillers are dispersed in a quantity of approximately 20-80% (w/w) and said at least one monolithic layer (9) comprises a polymer matrix in which mineral fillers are dispersed in a quantity of approximately 0-15% (w/w).

2. The multilayer sheet (1) according to claim 1, wherein said mineral fillers are dispersed in a quantity of 40-80% (w/w), preferably 50-75% (w/w) and even more preferably 65-75% (w/w).

3. The multilayer sheet (1) according to claim 1 or 2, wherein said multilayer sheet (1) has an outer face A which is represented by said monolithic layer (9) and an outer face B which is represented by said microporous layer (8).

4. The multilayer sheet (1) according to any one of claims 1 to 3, wherein said monolithic layer (9) comprises a polymer matrix in which are dispersed mineral fillers in a concentration of about 10-15% (w/w).

5. The multilayer sheet (1) according to any one of the claims 1 to 4, wherein said polymer matrix consists of polyolefins, selected for example from linear low-density polyethylene (LLDPE), low-density polyethylene (LDPE), high-density polyethylene (HDPE), polypropylene, or mixtures thereof, for example polyethylene, polypropylene, ethylene-propylene copolymers.

6. The multilayer sheet (1) according to any one of claims 1 to 5, where said mineral fillers are selected from the group which comprises calcium carbonate, talc, zeolites, kaolin, silica, magnesium carbonate, barium carbonate, magnesium sulfate, barium sulfate, calcium sulfate, aluminum hydroxide, magnesium hydroxide, zinc oxide, alumina, mica; or mixtures thereof.

7. The multilayer sheet (1) according to any of claims 1 to 6, wherein said mineral fillers are particles having an average particle size between 1 and 5 microns.

8. A film (10) for constructing a pouch (20), comprising the multilayer sheet (10) according to any one of claims 1 to 6 and an additional layer represented by a layer of nonstick material (5) applied to said monolithic layer (9).

9. The film (10) according to the preceding claim, wherein said nonstick material (5) is represented by silicone.

10. The film (10) according to claim 8 or 9, folded on itself so as to form a pouch (20), wherein the inner side A of said pouch (7) is represented by the microporous layer (8) of said multilayer sheet (1) and the inner side B of said pouch (20) is represented by the monolithic layer (9) of said multilayer sheet (1) .

11. A process for the preparation of a multilayer sheet (1) according to any one of claims 1 to 8, wherein said process comprises the steps of:
a) providing at least a first polymer matrix for the preparation of a microporous layer (8) comprising a polymer matrix in which mineral fillers are dispersed in a quantity of about 20-80% (w/w) and at least a second polymer matrix for the preparation of a monolithic layer (9) comprising a polymer matrix in which mineral fillers are dispersed in a quantity of about 0-15% (w/w);
b) coextruding said first and said second polymer matrix in the form of monolayers forming said multilayer sheet (1), wherein said multilayer sheet (1) is obtained by combining different hot layers inside the extruder.

12. The process according to claim 11, wherein said process also comprises a stretching step of said first polymer matrix.

13. The process according to claim 12, wherein said step of stretching said first polymer matrix takes place in the longitudinal direction.

14. A process for preparing a siliconized film (10) comprising the preparation of a multilayer sheet (1) according to the process of any one of claims 11 to 13, further comprising the step of applying on the outer face A, represented by the monolithic layer (9) of said multilayer sheet (1), a layer of a nonstick material (5).

15. A packaging made with the multilayer sheet (1) according to any one of claims 1 to 7 or with the film (10) according to any one of claims 8 to 10.

16. A packaging according to the preceding claim represented by a sachet or an envelope or a pouch (20).

17. A use of the multilayer material (1) according to any one of claims 1 to 7 or of the film (10) according to any one of claims 8 to 10 for food packaging.

18. A use of the multilayer material (1) according to any one of the claims 1 to 7 or of the film (10) according to any one of the claims 8 to 10 as a sheet for writing and/or printing.
